# FASCICULE DE BREVET EUROPEEN

(11) **EP 1 305 273 B1**
(45) Date de publication et mention de la délivrance du brevet: **27.04.2005**
(21) Numéro de dépôt: 01947587.0
(22) Date de dépôt: 22.06.2001
(51) Int. Cl.: C07C 51/31, C07C 55/02, C07C 55/14, B01J 23/84, B01J 23/86, B01J 23/889

(54) **PROCEDE D'OXYDATION D'HYDROCARBURES, D'ALCOOLS ET/OU DE CETONES**
VERFAHREN ZUR OXIDATION VON KOHLENWASSERSTOFFEN, ALKOHOLEN UND/ODER KETONEN
METHOD FOR OXIDISING HYDROCARBONS, ALCOHOLS AND/OR KETONES

(30) Priorité: 28.06.2000 FR 0008323
(43) Date de publication de la demande: 02.05.2003
(73) Titulaire: Rhodia Polyamide Intermediates, 69192 Saint-Fons (FR)
(72) Inventeur: FACHE, Eric, F-69300 Caluire et Cuire (FR)
(74) Mandataire: Esson, Jean-Pierre
(86) Numéro de dépôt international: PCT/FR2001/001976
(87) Numéro de publication internationale: WO 2002/000588

(56) Documents cités:
- EP-A- 0 784 045
- EP-A- 0 870 751
- WO-A-00/15598
- FR-A- 2 775 685

## Description

La présente invention concerne l'oxydation par l'oxygène ou un gaz en contenant, d'hydrocarbures en acides carboxyliques, alcools et/ou cétones correspondants ou d'alcools et/ou de cétones en acides carboxyliques correspondants.

L'oxydation directe par l'oxygène des hydrocarbures, plus particulièrement des cycloalcanes, en présence d'un catalyseur, est un procédé qui a été étudié depuis longtemps. En effet, il y aurait des avantages évidents à éviter l'emploi d'un oxydant comme l'acide nitrique, utilisé dans une des étapes des procédés industriels actuels, ce qui épargnerait le traitement des oxydes d'azote générés.

Dans les nombreuses variantes d'un tel procédé d'oxydation catalytique par l'oxygène, le cobalt est le catalyseur le plus fréquemment préconisé.

Ainsi le brevet américain US-A-2 223 493, publié en décembre 1940, décrit l'oxydation d'hydrocarbures cycliques en diacides correspondants, en phase liquide comportant généralement de l'acide acétique, à une température d'au moins 60°C, à l'aide d'un gaz contenant de l'oxygène et en présence d'un catalyseur d'oxydation tel qu'un composé du cobalt.

Le brevet américain US-A-4 902 827, publié en février 1990, décrit un perfectionnement à l'oxydation par l'air du cyclohexane en acide adipique, en phase liquide comportant l'acide acétique, à une température de 80°C à 160°C et en présence d'un catalyseur d'oxydation comprenant un composé soluble du cobalt et un composé soluble du zirconium et/ou de l'hafnium.

Plus récemment, il a été préconisé dans le brevet EP-A-0 694 333 de mettre en oeuvre dans le cadre de l'oxydation des hydrocarbures par l'oxygène, un catalyseur comprenant un sel cobaltique et un sel ferrique.

II a également était préconisé dans le brevet EP0870751 d'utiliser un catalyseur comprenant un sel cobaltique et un sel de chrome pour améliorer la sélectivité.

Comme autre catalyseur usuel de cette réaction d'oxydation, on peut citer le manganèse.

Pour des raisons économiques et également pour faciliter la purification des produits obtenus, il est préférable de travailler avec la concentration en catalyseur la plus faible possible. Ainsi, le manganèse est un catalyseur intéressant dans les procédés d'oxydation du cyclohexane.

II s'avère néanmoins que si les sélectivités obtenues avec les systèmes catalytiques utilisés dans les procédés antérieurs décrits ci-dessus sont acceptables, la productivité de ceux-ci doit être encore améliorée pour permettre une exploitation industrielle d'une telle réaction.

C'est ce que se propose de réaliser la présente invention. Elle consiste plus précisément en un procédé d'oxydation d'hydrocarbure, d'alcool et/ou de cétone à l'aide d'oxygène ou d'un gaz en contenant, en phase liquide et en présence d'un catalyseur dissous dans le milieu réactionnel, caractérisé en ce que le catalyseur comprend au moins un composé soluble du manganèse et/ou du cobalt, au moins un composé soluble du chrome et au moins un composé soluble du fer.

Les hydrocarbures qui sont utilisés comme substrats de départ dans le procédé de l'invention sont plus particulièrement les alcanes, les cycloalcanes, les hydrocarbures alkyl-aromatiques, les alcènes et les cycloalcènes ayant de 3 à 20 atomes de carbone.

Parmi ces hydrocarbures, les cycloalcanes, notamment ceux qui ont un cycle ayant de 5 à 12 atomes de carbone, sont certainement les plus importants, car leur oxydation conduit aux diacides carboxyliques ou aux cycloalcanols et cycloalcanones intermédiaires.

L'hydrocarbure le plus intéressant est le cyclohexane dont l'oxydation conduit à l'acide adipique, l'un des composés de base du polyamide 6-6, mais peut aussi fournir la cyclohexanone conduisant au caprolactame et donc au polyamide 6.

Le présent procédé peut également être utilisé pour l'oxydation des alcools ou cétones intermédiaires, notamment les cycloalcanols et cyclohexanones ayant de 5 à 12 atomes de carbone, pour préparer les diacides carboxyliques correspondants.
Dans ce qui suit, le procédé sera plus particulièrement décrit pour l'oxydation des hydrocarbures, essentiellement des cycloalcanes, et de manière privilégiée pour l'oxydation du cyclohexane.

Le système catalytique comprenant des composés du manganèse et/ou du cobalt, du chrome et du fer permet de préparer directement l'acide adipique avec une bonne sélectivité et une productivité améliorée, lorsque l'on réalise l'oxydation du cyclohexane. Ces propriétés catalytiques sont évidemment très avantageuses pour une exploitation industrielle de cette réaction d'oxydation.

Le système catalytique comprend soit au moins un composé du manganèse soluble dans le milieu réactionnel, choisi par exemple de manière non limitative parmi le chlorure de manganèse, le bromure de manganèse, le nitrate de manganèse et les carboxylates de manganèse comme l'acétate de manganèse tétrahydraté, le propionate de manganèse, l'adipate de manganèse, le glutarate de manganèse, le succinate de manganèse, soit au moins un composé du cobalt soluble dans le milieu réactionnel, choisi par exemple de manière non limitative parmi le chlorure de cobalt, le bromure de cobalt, le nitrate de cobalt et les carboxylates de cobalt comme l'acétate de cobalt tétrahydraté, le propionate de cobalt, l'adipate de cobalt, le glutarate de cobalt, le succinate de cobalt.

Le catalyseur comprend également au moins un composé du chrome soluble dans le milieu réactionnel, choisi par exemple de manière non limitative parmi le chlorure de chrome, le bromure de chrome, le nitrate de chrome et les carboxylates de chrome comme l'acétate de chrome, le propionate de chrome, l'adipate de chrome, le glutarate de chrome, le succinate de chrome, des sels minéraux ou organiques de l'acide chromique.

Le catalyseur comprend également au moins un composé du fer soluble dans le milieu réactionel, choisi par exemple de manière non limitative parmi les halogénures de fer, le nitrate de fer, les carboxylates de fer comme l'acétate, propionate, succinate, glutarate, adipate, les chélates de fer comme les acétylacétonates de fer.

Enfin, Le catalyseur peut comprendre également au moins un composé du zirconium et/ou de l'hafnium soluble dans le milieu réactionnel, choisi par exemple de manière non limitative parmi le chlorure de zirconium, le bromure de zirconium, le nitrate de zirconium et les carboxylates de zirconium comme l'acétate de zirconium, le propionate de zirconium, l'adipate de zirconium, le glutarate de zirconium, le succinate de zirconium et le chlorure d'hafnium, le bromure d'hafnium, le nitrate d'hafnium et les carboxylates d'hafnium comme l'acétate d'hafnium, le propionate d'hafnium, l'adipate d'hafnium, le glutarate d'hafnium, le succinate d'hafnium.

Les rapports molaires entre le manganèse et/ou le cobalt, le chrome et le fer dans le système catalytique peuvent varier dans de larges limites. On peut ainsi mettre en oeuvre des rapports molaires Mn et/ou Co / Cr / Fe compris avantageusement entre 1/0,00001/0,0001 à 1/100/100, préférentiellement entre 1/0,001/0,01 à 1/10/10.

La quantité de zirconium ou d'hafnium quand ils sont présents, peut varier dans des rapports molaires par rapport au manganèse ou au cobalt, semblables à ceux indiqués ci-dessus pour le chrome.

Le catalyseur peut être obtenu in situ en chargeant les composés du manganèse et/ou du cobalt, du chrome, du fer et éventuellement, du zirconium ou de l'hafnium dans le milieu réactionnel. Il peut également être préparé extemporanément par mélange desdits composés dans les proportions nécessaires pour obtenir les rapports molaires Mn et/ou Co/Cr/Fe et éventuellement Zr et/ou Hf souhaités. De préférence ce mélange est réalisé en utilisant un solvant, avantageusement un solvant de même nature que celui utilisé pour la réaction d'oxydation ou directement dans ce solvant.

La quantité de catalyseur, exprimée en pourcentage pondéral d'éléments métalliques (manganèse, cobalt, chrome, fer et éventuellement, zirconium ou hafnium) par rapport au mélange réactionnel, se situe généralement entre 0,0001 et 5 %, avantageusement entre 0,001 et 2 %, sans que ces valeurs soient critiques. II s'agit cependant d'avoir une activité suffisante sans toutefois utiliser des quantités trop importantes. En effet, le catalyseur devra être séparé du milieu réactionnel final et recyclé.

II est avantageux de mettre en oeuvre également un composé initiateur de la réaction d'oxydation. Les initiateurs sont souvent des hydroperoxydes, par exemple l'hydroperoxyde de cyclohexyle ou l'hydroperoxyde de tertiobutyle. Ce sont également des cétones ou des aldéhydes, par exemple la cyclohexanone qui est l'un des composés formés lors de l'oxydation du cyclohexane ou l'acétaldéhyde. Généralement l'initiateur représente de 0,01 % à 20 % en poids du poids du mélange réactionnel mis en oeuvre, sans que ces proportions aient une valeur critique. L'initiateur est surtout utile lors du démarrage de l'oxydation et lorsque l'on réalise l'oxydation du cyclohexane à une température inférieure à 120°C. Il peut être introduit dès le début de la réaction.

Le milieu réactionnel liquide contient préférentiellement un solvant au moins partiel de l'acide carboxylique et/ou de l'alcool et/ou de la cétone dont la préparation est visée par la mise en oeuvre du procédé de l'invention. Ce solvant peut être de nature très variée dans la mesure où il n'est pas sensiblement oxydable dans les conditions réactionnelles. II peut être notamment choisi parmi les solvants protiques polaires et les solvants aprotiques polaires. Comme solvants protiques polaires, on peut citer par exemple les acides carboxyliques ne possédant que des atomes d'hydrogène primaires ou secondaires, en particulier les acides aliphatiques ayant de 2 à 9 atomes de carbone, les acides perfluoroalkylcarboxyliques tel que l'acide trifluoroacétique, les alcools tels que le tertiobutanol. Comme solvants aprotiques polaires, on peut citer par exemple les esters d'alkyle inférieur (= radical alkyle ayant de 1 à 4 atomes de carbone) d'acides carboxyliques, en particulier des acides carboxyliques aliphatiques ayant de 2 à 9 atomes de carbone ou des acides perfluoroalkylcarboxyliques, la tétraméthylène sulfone (ou sulfolane), l'acétonitrile, les hydrocarbures halogénés tel que le dichlorométhane, les cétones telles que l'acétone.

L'acide acétique est utilisé de préférence comme solvant de la réaction d'oxydation du cyclohexane. Il est commode de mettre en oeuvre un catalyseur dont les constituants manganèse et chrome soient sous la forme de composés dérivant de l'acide carboxylique utilisé comme solvant, dans la mesure où lesdits composés sont solubles dans le milieu réactionnel. Les acétates de manganèse, de chrome et de fer sont donc utilisés de préférence, notamment pour cette raison.

Le solvant, tel que défini précédemment, représente généralement de 1 % à 99 % en poids du milieu réactionnel, de préférence de 10 % à 90 % et encore plus préférentiellement de 20 % à 80 %.

L'oxydation peut également être mise en oeuvre en présence d'eau introduite dès le stade initial du procédé.

La température à laquelle est réalisée la réaction d'oxydation est variable, notamment selon le substrat mis en oeuvre. Elle est généralement comprise entre 50°C et 200°C et de préférence entre 80 °C et 140°C.

La pression n'est pas un paramètre critique du procédé. Elle peut être inférieure, égale ou supérieure à la pression atmosphérique. Généralement elle se situera entre 0,1 MPa (1 bar) et 20 MPa (200 bar), sans que ces valeurs soient impératives.

On peut utiliser de l'oxygène pur, de l'air, de l'air enrichi ou appauvri en oxygène ou encore de l'oxygène dilué par un gaz inerte.

Les exemples qui suivent illustrent l'invention.

### EXEMPLE 1

Dans un autoclave de 1,5 I en titane, muni de moyens de chauffage par collier chauffant, de moyens de refroidissement, d'une turbine et de moyens d'introduction et de soutirage de gaz et de régulation de pression, préalablement purgé avec de l'azote, on charge :
- 292,5 g de cyclohexane
- 357 g d'acide acétique
- 3,4 g de cyclohexanone
- 4,16 g (16,7 mmol de Co) d'acétate de Cobalt tétrahydraté
- 0,162 g (0,74 mmol de Cr) d'acétate de chrome bis hydraté
- 1,183 g (3,2 mmol de Fe) d'acétyl acétonate de fer
- 0,8 g d'eau

Après fermeture du réacteur, on agite à 1000 tours par minute, on crée une pression d'azote de 20 bar à 20°C et on chauffe. La température atteint 105°C dans la masse en 20 min. L'azote est remplacé par de l'air à 5 % d'oxygène sous une pression de 20 bar. Le débit normal gazeux de l'air est de 250 l/h. Après une brève période de l'ordre de quelques minutes sans consommation d'oxygène, la température s'élève de quelques degrés et une consommation d'oxygène est observée. La teneur en oxygène dans l'air est progressivement augmentée jusqu'à la valeur de 21 %. La concentration en oxygène dans le gaz sortant du réacteur reste inférieure à 5 %.
Après 76 minutes de réaction, 52,8 normal litres d'oxygène ont été consommés, correspondant à un taux de conversion du cyclohexane d'environ 20 %.

Après arrêt du balayage d'air et refroidissement à une température de 70°C, le mélange réactionnel est analysé pour déterminer le taux de conversion et la sélectivité. Ces analyses sont réalisées par chromatographie en phase gazeuse (par sélectivité-ST-on entend le rapport molaire exprimé en pour cent du nombre de moles dosées d'une espèce par rapport au nombre de moles théorique de l'espèce calculé à partir du nombre de moles de cyclohexane effectivement transformé) :.

On obtient les résultats suivants :
- taux de transformation (TT) du cyclohexane : 20,7 %
- ST en cyclohexanol par rapport au cyclohexane transformé : 6,3 %
- ST en cyclohexanone par rapport au cyclohexane transformé : 4,9 %
- ST en acide adipique par rapport au cyclohexane transformé : 67,1 %
- ST en acide adipique + cyclohexanone + cyclohexanol par rapport au cyclohexane transformé : 78,3 %
- rapport molaire acide adipique/total des diacides formés : 85,9 %

### EXEMPLE 2 COMPARATIF

On répète l'exemple 1 dans le même appareillage et dans les mêmes conditions opératoires, avec la charge de réactifs suivante :
- 292,5 g de cyclohexane
- 357 g d'acide acétique
- 3,4 g de cyclohexanone
- 4,0 (16,2 mmol de Co) d'acétate de Cobalt tétrahydraté
- 0,157 g (0,64 mmol de Cr) d'acétate de chrome bis hydraté
- 0,6 g d'eau
Le temps de réaction est de 95 minutes au lieu de 76 minutes dans l'exemple 1, pour un taux de transformation équivalent.

On obtient les résultats suivants :
- taux de transformation (TT) du cyclohexane : 21,1 %
- ST en cyclohexanol par rapport au cyclohexane transformé : 5,1 %
- ST en cyclohexanone par rapport au cyclohexane transformé : 3,4 %
- ST en acide adipique par rapport au cyclohexane transformé : 70,9 %
- ST en acide adipique + cyclohexanone + cyclohexanol par rapport au cyclohexane transformé : 79,4 %
- rapport molaire acide adipique/total des diacides formés : 85,7 %

Cet essai montre clairement l'influence du fer sur l'activité du catalyseur. En effet, pour obtenir un taux de transformation semblable du cyclohexane, le temps de réaction a été diminué de 25 % dans l'exemple 1, en conservant une sélectivité en acide adipique équivalente.

### EXEMPLE 3 COMPARATIF

On répète l'exemple 1 dans le même appareillage et dans les mêmes conditions opératoires, mais en ajoutant la charge suivante :
- 292,5 g de cyclohexane
- 357 g d'acide acétique
- 3,4 g de cyclohexanone
- 4,17 g (16,7 mmol de Co) d'acétate de Cobalt tétrahydraté
- 0,8 g d'eau
Le temps de réaction est de 75 minutes.
Les résultats obtenus sont :
- taux de transformation (TT) du cyclohexane : 20,3 %
- ST en cyclohexanol par rapport au cyclohexane transformé : 11,6 %
- ST en cyclohexanone par rapport au cyclohexane transformé : 4,5 %
- ST en acide adipique par rapport au cyclohexane transformé : 61,9 %
- ST en acide adipique + cyclohexanone + cyclohexanol par rapport au cyclohexane transformé : 78,2 %
- rapport molaire acide adipique/total des diacides formés : 85,4 %

Cet essai montre, en comparaison avec l'exemple 1, l'effet positif de la présence du fer et du chrome sur la sélectivité et la productivité du catalyseur

### EXEMPLE 4

On répète l'exemple 1 dans le même appareillage et dans les mêmes conditions opératoires, mais en chargeant dans le réacteur les composés suivants :
- 292,5 g de cyclohexane
- 357 g d'acide acétique
- 3,4 g de cyclohexanone
- 4,13 g (16,6 mmol de Co) d'acétate de Cobalt tétrahydraté
- 0,2325 g (1,06 mmol de Cr) d'acétate de chrome bis hydraté
- 1,086 g (3,1 mmol de Fe) d'acétyl acétonate de fer
- 0,8 g d'eau
Le temps de la réaction est de 73 minutes.

On obtient les résultats suivants :
- taux de transformation (TT) du cyclohexane : 20,3 %
- ST en cyclohexanol par rapport au cyclohexane transformé : 9,8 %
- ST en cyclohexanone par rapport au cyclohexane transformé : 2,5 %
- ST en acide adipique par rapport au cyclohexane transformé : 68,8 %
- ST en acide adipique + cyclohexanone + cyclohexanol par rapport au cyclohexane transformé : 78,3 %
- rapport molaire acide adipique/total des diacides formés : 85,3 %

### EXEMPLE 5 COMPARATIF

On répète l'exemple 1 dans le même appareillage et dans les mêmes conditions opératoires, mais en chargeant dans le réacteur les composés suivants :
- 292,5 g de cyclohexane
- 357 g d'acide acétique
- 3,4 g de cyclohexanone
- 4,0 g (16,1 mmol de Co) d'acétate de Cobalt tétrahydraté
- 0,309 g (1,25 mmol de Cr) d'acétate de chrome bis hydraté
- 0,6 g d'eau
Le temps d'induction de la réaction est de 50 minutes et le temps de la réaction est de 160 minutes.

On obtient les résultats suivants :
- taux de transformation (TT) du cyclohexane : 17 %
- ST en cyclohexanol par rapport au cyclohexane transformé : 4,6 %
- ST en cyclohexanone par rapport au cyclohexane transformé : 1,7 %
- ST en acide adipique par rapport au cyclohexane transformé : 74,3 %
- ST en acide adipique + cyclohexanone + cyclohexanol par rapport au cyclohexane transformé : 77,2 %
- rapport molaire acide adipique/total des diacides formés : 83,4 %

Cet essai pris en comparaison avec l'exemple 4 montre clairement l'effet positif sur la productivité de l'association du fer avec le chrome, sans affecter significativement la sélectivité.

### EXEMPLE 6

Dans un autoclave de 1,5 I en titane, muni de moyens de chauffage par collier chauffant, de moyens de refroidissement, d'une turbine et de moyens d'introduction et de soutirage de gaz et de régulation de pression, préalablement purgé avec de l'azote, on charge :
- 292,5 g de cyclohexane
- 357 g d'acide acétique
- 3,67g de cyclohexanone
- 4,13 g (16,6 mmol de Co) d'acétate de Cobalt tétrahydraté
- 0,1595 g (0,73 mmol de Cr) d'acétate de chrome bis hydraté
- 1,0895 g (3,1 mmol de Fe) d'acétyl acétonate de fer
- 0,8 g d'eau

Après fermeture du réacteur, on agite à 1000 tours par minute. On crée une pression d'azote de 20 bar à 20°C et on chauffe. La température atteint 105°C dans la masse en 20 min. L'azote est remplacé par de l'air à 5 % d'oxygène sous une pression de 20 bar. Le débit normal gazeux de l'air est de 250 l/h. Après une brève période de l'ordre de quelques minutes sans consommation d'oxygène, la température s'élève de quelques degrés et une consommation d'oxygène est observée. La teneur en oxygène dans l'air est progressivement augmenté jusqu'à la valeur de 21 %. La concentration en oxygène dans le gaz sortant du réacteur reste inférieure à 5 %.
Quand 50 normal litres d'oxygène ont été consommés, correspondant à un taux de conversion du cyclohexane d'environ 20 %, on commence l'injection en continu dans la phase liquide d'une solution d'acide acétique contenant 1,1 % en poids d'acétate de cobalt tétrahydraté, 0,043 % en poids d'acétate de chrome bis hydraté et 0,3% en poids d'acétyl acétonate de fer à un débit de 3,9 ml/min et une injection de 4,3 ml/min de cyclohexane.
La consommation d'oxygène pendant la période d'injection est de 0,6 l/min.

Après arrêt du balayage d'air et arrêt des injections de réactifs, le mélange est refroidi à une température de 70°C. Le mélange réactionnel est analysé pour déterminer les différents taux de conversion et la sélectivité. Ces analyses sont réalisées par chromatographie en phase gazeuse.

On obtient les résultats suivants :
- taux de transformation (TT) du cyclohexane : 19,6 %
- ST en cyclohexanol par rapport au cyclohexane transformé : 6,5 %
- ST en cyclohexanone par rapport au cyclohexane transformé : 6,0 %
- ST en acide adipique par rapport au cyclohexane transformé : 65,3 %
- ST en acide adipique + cyclohexanone + cyclohexanol par rapport au cyclohexane transformé : 77,8 %
- rapport molaire acide adipique/total des diacides formés : 85,1 %

La productivité du catalyseur est de 60,7 g d'acide adipique produit par litre et heure.

### EXEMPLE 7 COMPARATIF

On répète l'exemple 6 dans le même appareillage et dans les mêmes conditions opératoires, mais en supprimant uniquement le fer dans la charge initiale et dans la solution injectée.
La consommation d'oxygène pendant la période d'injection est de 0,44 l/min.

On obtient les résultats suivants :
- taux de transformation (TT) du cyclohexane : 18,2 %
- ST en cyclohexanol par rapport au cyclohexane transformé : 5,1 %
- ST en cyclohexanone par rapport au cyclohexane transformé : 4,8 %
- ST en acide adipique par rapport au cyclohexane transformé : 69,5 %
- ST en acide adipique + cyclohexanone + cyclohexanol par rapport au cyclohexane transformé : 79,4 %
- rapport molaire acide adipique/total des diacides formés : 85,0 %.

La productivité du catalyseur est de 47,5 g d'acide adipique par litre et heure.

### EXEMPLE 8 COMPARATIF

On répète l'exemple 7 dans le même appareillage et dans les mêmes conditions opératoires, mais en supprimant le fer et le chrome dans la charge initiale et dans la solution injectée. La consommation d'oxygène pendant la période d'injection est de 0,55 l/min.

On obtient les résultats suivants :
- taux de transformation (TT) du cyclohexane : 18,5 %
- ST en cyclohexanol par rapport au cyclohexane transformé : 10,8 %
- ST en cyclohexanone par rapport au cyclohexane transformé : 5,8 %
- ST en acide adipique par rapport au cyclohexane transformé : 61,6 %
- ST en acide adipique + cyclohexanone + cyclohexanol par rapport au cyclohexane transformé : 78,2 %
- rapport molaire acide adipique/total des diacides formés : 84,0 %
La productivité du catalyseur est de 56,5 g d'acide adipique par litre et heure.

### Exemple 9 comparatif

Dans un autoclave de 125 ml en titane, muni de moyens de chauffage par collier chauffant, d'une turbine et de moyens d'introduction de gaz et de régulation de pression, on charge :
- 21,25 g (253 mmol) de cyclohexane
- 27,35 g d'acide acétique
- 0,26 g (2,65 mmol) de cyclohexanone
- 0,0357 g (0,146 mmol de Mn) d'acétate de manganèse tétrahydraté
- 0,011 g d'acétate de chrome bis hydraté(0,04 mmol de Cr).

Après fermeture du réacteur, on agite à 1000 tours par minute, on crée une pression d'air (100 bar à 20°C) et on chauffe. La température atteint 105°C dans la masse en 10 min et on maintient cette température pendant encore 150 min.

Après refroidissement et dépressurisation, le mélange réactionnel est constitué de deux phases liquides que l'on homogénéise par addition d'acide acétique.

Le mélange homogène ainsi obtenu est dosé par chromatographie en phase gazeuse.

On obtient les résultats suivants :
- taux de transformation (TT) du cyclohexane : 14,9 %
- ST en cyclohexanol par rapport au cyclohexane transformé : 19,4 %
- ST en cyclohexanone par rapport au cyclohexane transformé : 0,0 %
- ST en acide adipique par rapport au cyclohexane transformé : 50 %
- ST en acide adipique + cyclohexanone + cyclohexanol par rapport au cyclohexane transformé : 69,4 %
- rapport molaire acide adipique/total des diacides formés : 77,6 %

### Exemple 10

L'exemple 9 est répété mais en utilisant comme système catalytique le système de composition suivante :
- 0,3107 g (1,247 mmol de Co) d'acétate de Cobalt tétrahydraté
- 0,0119 g (0,012 mmol de Cr) d'acétate de chrome bis hydraté
- 0,0861 g (0,244 mmol de Fe) d'acétyl acétonate de fer
- 0,0525 g (0,149 mmol de Mn) d'acétylacétonate de manganèse (III)
Le mélange est maintenu à 105°C pendant 45 minutes.

On obtient les résultats suivants :
- taux de transformation (TT) du cyclohexane : 12,1 %
- ST en cyclohexanol par rapport au cyclohexane transformé : 9,5 %
- ST en cyclohexanone par rapport au cyclohexane transformé : 8,1 %
- ST en acide adipique par rapport au cyclohexane transformé : 67 %
- ST en acide adipique + cyclohexanone + cyclohexanol par rapport au cyclohexane transformé : 85,6 %
- rapport molaire acide adipique/total des diacides formés : 85,5 %

### Exemple 11

L'exemple 10 est répété mais en utilisant comme système catalytique le système de composition suivante :
- 0,3135 g (1,258 mmol de Co) d'acétate de Cobalt tétrahydraté
- 0,0114 g (0,0113 mmol de Cr) d'acétate de chrome bis hydraté
- 0,0828 g (0,234 mmol de Fe) d'acétyl acétonate de fer
- 0,0522 g (0,148 mmol de Mn) d'acétylacétonate de manganèse (III)
- 0,0059 g (0,0121 mmol de Zr) d'acétylacétonate de zirconium

On obtient les résultats suivants :
- taux de transformation (TT) du cyclohexane : 11,7 %
- ST en cyclohexanol par rapport au cyclohexane transformé : 8,8 %
- ST en cyclohexanone par rapport au cyclohexane transformé : 9,4 %
- ST en acide adipique par rapport au cyclohexane transformé : 67,4 %
- ST en acide adipique + cyclohexanone + cyclohexanol par rapport au cyclohexane transformé : 85,6 %
- rapport molaire acide adipique/total des diacides formés : 85,7 %

## Revendications

1. Procédé d'oxydation d'hydrocarbures, d'alcools et/ou de cétones en acide carboxylique, à l'aide d'oxygène ou d'un gaz en contenant, en phase liquide dans un solvant choisi parmi les solvants protiques polaires et les solvants aprotiques polaires et en présence d'un catalyseur dissous dans le milieu réactionnel, **caractérisé en ce que** le catalyseur comprend un composé soluble du manganèse et/ou du cobalt, un composé soluble du chrome, et un composé soluble du fer.

2. Procédé selon la revendication 1, **caractérisé en ce que** l'hydrocarbure utilisé comme substrat de départ est choisi parmi les cycloalcanes qui ont un cycle ayant de 5 à 12 atomes de carbone, et est de préférence le cyclohexane.

3. Procédé selon la revendication 1, **caractérisé en ce que** l'alcool et/ou la cétone utilisés comme substrat de départ sont choisis parmi les cycloalcanols et cycloalcanones qui ont un cycle ayant de 5 à 12 atomes de carbone, et sont de préférence le cyclohexanol et/ou la cyclohexanone.

4. Procédé selon l'une des revendications 1 à 3, **caractérisé en ce que** le catalyseur comprend au moins un composé du manganèse soluble dans le milieu réactionnel, choisi parmi le chlorure de manganèse, le bromure de manganèse, le nitrate de manganèse et les carboxylates de manganèse comme l'acétate de manganèse tétrahydraté, le propionate de manganèse, l'adipate de manganèse, le glutarate de manganèse, le succinate de manganèse.

5. Procédé selon l'une des revendications 1 à 4, **caractérisé en ce que** le catalyseur comprend au moins un composé du cobalt soluble dans le milieu réactionnel, choisi parmi le chlorure de cobalt, le bromure de cobalt, le nitrate de cobalt et les carboxylates de cobalt comme l'acétate de cobalt, le propionate de cobalt, l'adipate de cobalt, le glutarate de cobalt, le succinate de cobalt.

6. Procédé selon l'une des revendications 1 à 5, **caractérisé en ce que** le catalyseur comprend au moins un composé du chrome soluble dans le milieu réactionnel, choisi parmi le chlorure de chrome, le bromure de chrome, le nitrate de chrome et les carboxylates de chrome comme l'acétate de chrome, le propionate de chrome, l'adipate de chrome, le glutarate de chrome, le succinate de chrome, les sels minéraux ou organiques de l'acide chromique.

7. Procédé selon l'une des revendications 1 à 6, **caractérisé en ce que** le catalyseur comprend au moins un composé du fer soluble dans le milieu réactionnel, choisi parmi le chlorure de fer, le bromure de fer, le nitrate de fer et les carboxylates de fer comme l'acétate de fer, le propionate de fer, l'adipate de fer, le glutarate de fer, le succinate de fer.

8. Procédé selon l'une des revendications précédentes, **caractérisé en ce que** le catalyseur comprend un composé soluble du zirconium ou d'hafnium

9. Procédé selon la revendication 8, **caractérisé en ce que** le composé soluble du zirconium dans le milieu réactionnel est choisi parmi le chlorure de zirconium, le bromure de zirconium, le nitrate de zirconium et les carboxylates de zirconium comme l'acétate de zirconium, le propionate de zirconium, l'adipate de zirconium, le glutarate de zirconium, le succinate de zirconium.

10. Procédé selon la revendication 8, **caractérisé en ce que** le composé soluble du hafnium dans le milieu réactionnel est choisi parmi le chlorure d'hafnium, le bromure d'hafnium, le nitrate d'hafnium et les carboxylates d'hafnium comme l'acétate d'hafnium, le propionate d'hafnium, l'adipate d'hafnium, le glutarate d'hafnium, le succinate d'hafnium.

11. Procédé selon l'une des revendications 1 à 10, **caractérisé en ce que** les rapports molaires entre le chrome, le manganèse ou le cobalt, le chrome et le fer est compris 1/0,00001/0,0001 et 1/100/100.

12. Procédé selon la revendication 11, **caractérisé en ce que** les rapports molaires entre le chrome, le manganèse ou le cobalt, le chrome et le fer est compris 1/0,001/0,001 et 1/10/10.

13. Procédé selon l'une des revendications 6 à 12, **caractérisé en ce que** la quantité de catalyseur, exprimée en pourcentage pondéral d'éléments métalliques par rapport au mélange réactionnel, se situe entre 0,0001 et 5 %, de préférence entre 0,001 et 2 %.

14. Procédé selon l'une des revendications 1 à 13, **caractérisé en ce que** le milieu réactionnel liquide contient un solvant choisi parmi les acides carboxyliques aliphatiques ayant de 2 à 9 atomes de carbone, les acides perfluoroalkylcarboxyliques, les alcools, les hydrocarbures halogénés, les cétones, les esters d'alkyle inférieur d'acides carboxyliques, de préférence des acides carboxyliques aliphatiques ayant de 2 à 9 atomes de carbone ou des acides perfluoroalkylcarboxyliques, la tétraméthylène sulfone (ou sulfolane), l'acétonitrile.

15. Procédé selon l'une des revendications 1 à 14, **caractérisé en ce que** le solvant utilisé est l'acide acétique.

16. Procédé selon l'une des revendications 1 à 15, **caractérisé en ce que** le solvant représente de 1 % à 99 % en poids du milieu réactionnel, de préférence de 10 % à 90.

17. Procédé selon l'une des revendications 1 à 16, **caractérisé en ce que** la température à laquelle est réalisée la réaction d'oxydation est comprise entre 50°C et 200°C et de préférence entre 80 °C et 140°C.

18. Procédé selon l'une des revendications 1 à 17, **caractérisé en ce que** la pression à laquelle est réalisée la réaction d'oxydation est comprise entre 0,1 MPa (1 bar) et 20 MPa (200 bar).

## Patentansprüche

1. Verfahren zur Oxidation von Kohlenwasserstoffen, Alkoholen und/oder Ketonen zu Carbonsäure mit Hilfe von Sauerstoff oder eines Gases, das Sauerstoff enthält, in flüssiger Phase in einem Lösungsmittel, das ausgewählt ist unter den polaren protischen Lösungsmitteln und den polaren aprotischen Lösungsmitteln, und in Gegenwart eines in dem Reaktionsmedium gelösten Katalysators, **dadurch gekennzeichnet, dass** der Katalysator eine lösliche Mangan- und/oder Kobaltverbindung, eine lösliche Chromverbindung und eine lösliche Eisenverbindung umfasst.

2. Verfahren gemäß Anspruch 1, **dadurch gekennzeichnet, dass** der als Ausgangssubstrat verwendete Kohlenwasserstoff ausgewählt ist unter den Cycloalkanen, die einen Ring mit 5 bis 12 Kohlenstoffatomen besitzen, und vorzugsweise Cyclohexan ist.

3. Verfahren gemäß Anspruch 1, **dadurch gekennzeichnet, dass** der Alkohol und/oder das Keton, die als Ausgangssubstrat verwendet werden, ausgewählt sind unter den Cycloalkanolen und Cycloalkanonen, die einen Ring mit 5 bis 12 Kohlenstoffatomen besitzen, und vorzugsweise Cyclohexanol und/oder Cyclohexanon sind.

4. Verfahren gemäß einem der Ansprüche 1 bis 3, **dadurch gekennzeichnet, dass** der Katalysator wenigstens eine in dem Reaktionsmedium lösliche Manganverbindung umfasst, die ausgewählt ist unter Manganchlorid, Manganbromid, Mangannitrat und den Mangancarboxylaten, wie Manganacetat Tetrahydrat, Manganpropionat, Manganadipat, Manganglutarat, Mangansuccinat.

5. Verfahren gemäß einem der Ansprüche 1 bis 4, **dadurch gekennzeichnet, dass** der Katalysator wenigstens eine in dem Reaktionsmedium lösliche Kobaltverbindung umfasst, die ausgewählt ist unter Kobaltchlorid, Kobaltbromid, Kobaltnitrat und den Kobaltcarboxylaten, wie Kobaltacetat, Kobaltpropionat, Kobaltadipat, Kobaltglutarat, Kobaltsuccinat.

6. Verfahren gemäß einem der Ansprüche 1 bis 5, **dadurch gekennzeichnet, dass** der Katalysator wenigstens eine in dem Reaktionsmedium lösliche Chromverbindung umfasst, die ausgewählt ist unter Chromchlorid, Chrombromid, Chromnitrat und den Chromcarboxylaten, wie Chromacetat, Chrompropionat, Chromadipat, Chromglutarat, Chromsuccinat, den mineralischen oder organischen Salzen von Chromsäure.

7. Verfahren gemäß einem der Ansprüche 1 bis 6, **dadurch gekennzeichnet, dass** der Katalysator wenigstens eine in dem Reaktionsmedium lösliche Eisenverbindung umfasst, die ausgewählt ist unter Eisenchlorid, Eisenbromid, Eisennitrat und den Eisencarboxylaten, wie Eisenacetat, Eisenpropionat, Eisenadipat, Eisenglutarat, Eisensuccinat.

8. Verfahren gemäß einem der vorhergehenden Ansprüche, **dadurch gekennzeichnet, dass** der Katalysator eine lösliche Zirkonium- oder Hafniumverbindung umfasst.

9. Verfahren gemäß Anspruch 8, **dadurch gekennzeichnet, dass** die in dem Reaktionsmedium lösliche Zirkoniumverbindung ausgewählt ist unter Zirkoniumchlorid, Zirkoniumbromid, Zirkoniumnitrat und den Zirkoniumcarboxylaten, wie Zirkoniumacetat, Zirkoniumpropionat, Zirkoniumadipat, Zirkoniumglutarat, Zirkoniumsuccinat.

10. Verfahren gemäß Anspruch 8, **dadurch gekennzeichnet, dass** die in dem Reaktionsmedium lösliche Hafniumverbindung ausgewählt ist unter Hafniumchlorid, Hafniumbromid, Hafniumnitrat und den Hafniumcarboxylaten, wie Hafniumacetat, Hafniumpropionat, Hafniumadipat, Hafniumglutarat, Hafniumsuccinat.

11. Verfahren gemäß einem der Ansprüche 1 bis 10, **dadurch gekennzeichnet, dass** die Molverhältnisse zwischen Chrom, Mangan oder Kobalt, Chrom und Eisen zwischen 1 / 0,00001 / 0,0001 und 1 / 100 / 100 liegen.

12. Verfahren gemäß Anspruch 11, **dadurch gekennzeichnet, dass** die Molverhältnisse zwischen Chrom, Mangan oder Kobalt, Chrom und Eisen zwischen 1 / 0,001 / 0,001 und 1 / 10 / 10 liegen.

13. Verfahren gemäß einem der Ansprüche 6 bis 12, **dadurch gekennzeichnet, dass** die Menge an Katalysator, ausgedrückt in Gewichtsprozent metallische Elemente bezogen auf das Reaktionsgemisch, zwischen 0,0001 und 5 %, vorzugsweise zwischen 0,001 und 2 % liegt.

14. Verfahren gemäß einem der Ansprüche 1 bis 13, **dadurch gekennzeichnet, dass** das flüssige Reaktionsmedium ein Lösungsmittel enthält, das ausgewählt ist unter den aliphatischen Carbonsäuren mit 2 bis 9 Kohlenstoffatomen, den Perfluoralkylcarbonsäuren, den Alkoholen, den halogenierten Kohlenwasserstoffen, den Ketonen, den niederen Alkylestern von Carbonsäuren, vorzugsweise der aliphatischen Carbonsäuren mit 2 bis 9 Kohlenstoffatomen oder der Perfluoralkylcarbonsäuren, Tetramethylensulfon (oder Sulfolan), Acetonitril.

15. Verfahren gemäß einem der Ansprüche 1 bis 14, **dadurch gekennzeichnet, dass** das verwendete Lösungsmittel. Essigsäure ist.

16. Verfahren gemäß einem der Ansprüche 1 bis 15, **dadurch gekennzeichnet, dass** das Lösungsmittel 1 bis 99 Gew.-% des Reaktionsmediums, vorzugsweise 10 bis 90 Gew.-% darstellt.

17. Verfahren gemäß einem der Ansprüche 1 bis 16, **dadurch gekennzeichnet, dass** die Temperatur, bei der die Oxidationsreaktion durchgeführt wird, zwischen 50 °C und 200 °C und vorzugsweise zwischen 80 °C und 140 °C liegt.

18. Verfahren gemäß einem der Ansprüche 1 bis 17, **dadurch gekennzeichnet, dass** der Druck, bei dem die Oxidationsreaktion durchgeführt wird, zwischen 0,1 MPa (1 bar) und 20 MPa (200 bar) liegt.

## Claims

1. Process for oxidizing hydrocarbons, alcohols and/or ketones to carboxylic acid using oxygen or a gas containing it, in a liquid phase in a solvent chosen from polar protic solvents and polar aprotic solvents and in the presence of a catalyst dissolved in the reaction medium, **characterized in that** the catalyst comprises a soluble manganese and/or cobalt compound, a soluble chromium compound and a soluble iron compound.

2. Process according to claim 1, **characterized in that** the hydrocarbon used as starting substrate is chosen from cycloalkanes which have a ring containing from 5 to 12 carbon atoms, and is preferably cyclohexane.

3. Process according to claim 1, **characterized in that** the alcohol and/or the ketone used as starting substrate are chosen from cycloalkanols and cycloalkanones which have a ring containing from 5 to 12 carbon atoms, and are preferably cyclohexanol and/or cyclohexanone.

4. Process according to one of claims 1 to 3, **characterized in that** the catalyst comprises at least one manganese compound which is soluble in the reaction medium, chosen from manganese chloride, manganese bromide, manganese nitrate and manganese carboxylates, for instance manganese acetate tetrahydrate, manganese propionate, manganese adipate, manganese glutarate and manganese succinate.

5. Process according to one of claims 1 to 4, **characterized in that** the catalyst comprises at least one cobalt compound which is soluble in the reaction medium, chosen from cobalt chloride, cobalt bromide, cobalt nitrate and cobalt carboxylates, for instance cobalt acetate, cobalt propionate, cobalt adipate, cobalt glutarate and cobalt succinate.

6. Process according to one of claims 1 to 5, **characterized in that** the catalyst comprises at least one chromium compound which is soluble in the reaction medium, chosen from chromium chloride, chromium bromide, chromium nitrate and chromium carboxylates, for instance chromium acetate, chromium propionate, chromium adipate, chromium glutarate, chromium succinate and mineral or organic chromic acid salts.

7. Process according to one of claims 1 to 6, **characterized in that** the catalyst comprises at least one iron compound which is soluble in the reaction medium, chosen from iron chloride, iron bromide, iron nitrate and iron carboxylates, for instance iron acetate, iron propionate, iron adipate, iron glutarate and iron succinate.

8. Process according to one of the preceding claims, **characterized in that** the catalyst comprises a soluble zirconium or hafnium compound.

9. Process according to claim 8, **characterized in that** the zirconium compound which is soluble in the reaction medium is chosen from zirconium chloride, zirconium bromide, zirconium nitrate and zirconium carboxylates, for instance zirconium acetate, zirconium propionate, zirconium adipate, zirconium glutarate or zirconium succinate.

10. Process according to claim 8, **characterized in that** the hafnium compound which is soluble in the reaction medium is chosen from hafnium chloride, hafnium bromide, hafnium nitrate and hafnium carboxylates, for instance hafnium acetate, hafnium propionate, hafnium adipate, hafnium glutarate or hafnium succinate.

11. Process according to one of claims 1 to 10, **characterized in that** the molar ratios between the chromium, the manganese or the cobalt, the chromium and the iron is between 1/0.00001/0.0001 and 1/100/100.

12. Process according to claim 11, **characterized in that** the molar ratios between the chromium, the manganese or the cobalt, the chromium and the iron is between 1/0.001/0.001 and 1/10/10.

13. Process according to one of claims 6 to 12, **characterized in that** the amount of catalyst, expressed as a weight percentage of metal elements relative to the reaction mixture, is between 0.0001% and 5% and preferably between 0.001% and 2%.

14. Process according to one of claims 1 to 13, **characterized in that** the liquid reaction medium contains a solvent chosen from aliphatic carboxylic acids containing from 2 to 9 carbon atoms, perfluoroalkylcarboxylic acids, alcohols, halogenated hydrocarbons, ketones, lower alkyl esters of carboxylic acids, preferably of aliphatic carboxylic acids containing from 2 to 9 carbon atoms or of perfluoroalkylcarboxylic acids, tetramethylene sulphone (or sulpholane) and acetonitrile.

15. Process according to one of claims 1 to 14, **characterized in that** the solvent used is acetic acid.

16. Process according to one of claims 1 to 15, **characterized in that** the solvent represents from 1% to 99% by weight of the reaction medium and preferably from 10% to 90.

17. Process according to one of claims 1 to 16, **characterized in that** the temperature at which the oxidation reaction is carried out is between 50°C and 200°C and preferably between 80°C and 140°C.

18. Process according to one of claims 1 to 17, **characterized in that** the pressure at which the oxidation reaction is carried out is between 0.1 MPa (1 bar) and 20 MPa (200 bar).
